# EUROPÄISCHE PATENTANMELDUNG

(11) **EP 0 968 996 A2**
(43) Veröffentlichungstag der Anmeldung: **05.01.2000**
(21) Anmeldenummer: 99110215.3
(22) Anmeldetag: 26.05.1999
(51) Int. Cl.: C07C 209/72, B01J 23/56

(54) **Verfahren zur Herstellung von Gemischen aus Cyclohexylamin und Dicyclohexylamin in variablen Mengenverhältnissen**

(30) Priorität: 04.06.1998 DE 19824906
(71) Anmelder: Bayer Aktiengesellschaft, 51368 Leverkusen (DE)
(72) Erfinder: Langer, Reinhard, Dr., 47918 Tönisvorst (DE); Petruck, Gerd-Michael,Dr., 40699 Erkrath (DE)

(57) **Zusammenfassung**

Die vorliegende Erfindung betrifft ein Hochdruckverfahren zur Hydrierung von aromatischen Aminen zu Gemischen aus den entsprechenden cycloaliphatischen Aminen und dicycloaliphatischen Aminen in variablen Mengenverhältnissen in Gegenwart von Rhodium-Katalysatoren die gegebenenfalls mit Ir, Ru, Os, Pd oder Pt oder Gemischen dieser Metalle modifiziert sind, auf mit Oxiden des Cr, Mo, W, Mn oder Re oder Gemischen dieser Oxide modifizierten Trägern.

## Beschreibung

Die vorliegende Erfindung betrifft ein Hochdruckverfahren zur Hydrierung von aromatischen Aminen zu Gemischen aus den entsprechenden cycloaliphatischen Aminen und dicycloaliphatischen Aminen in variablen Mengenverhältnissen in Gegenwart von Rhodium-Katalysatoren die gegebenenfalls mit einem Edelmetall der Reihe Ir, Ru, Os, Pd oder Pt oder Gemischen dieser Metalle modifiziert sind, auf mit Oxiden des Cr, Mo, W, Mn oder Re oder Gemischen dieser Oxide modifizierten Trägern.

Gegebenenfalls substituierte Cyclohexylamine und Dicyclohexylamine finden Verwendung zur Herstellung von Alterungsschutzmitteln für Kautschuke und Kunststoffe, als Korrosionsschutzmittel in wäßriger Lösung sowie als Vorprodukte für Textilhilfsmittel und Pflanzenschutzmittel.

Es ist bekannt, Cyclohexylamin durch Druckhydrierung von Anilin herzustellen. Für diese Hydrierung werden hauptsächlich Edelmetall-Katalysatoren eingesetzt, beispielsweise gemäß US 3.636.108 ein mit Alkali moderierter Ru-Katalysator, wobei noch zusätzlich NH₃ und gegebenenfalls ein Lösungsmittel eingesetzt werden. Ein weiteres Verfahren zur Druckhydrierung von Anilin zu Cyclohexylamin ist in DE-B 1 106 319 beschrieben, wobei ebenfalls ein Ru-Katalysator verwendet wird. In diesem Verfahren wird mitentstehendes Dicyclohexylamin dem Einsatzstoff wieder zugesetzt. Das Verfahren erreicht jedoch wegen der gleichzeitigen Entstehung von Cyclohexan nur eine mäßige Ausbeute. Nach EP-B 0 053 818 sind Pd-Trägerkatalysatoren günstiger als Ru-Katalysatoren; die dort beschriebenen Katalysatoren enthalten Zusätze, die entweder einer Gruppe von basischen Verbindungen der Alkalimetalle, Erdalkalimetalle und Seltenerdmetalle entstammen oder einer Gruppe, welche die Metalle Fe, Ni, Co, Mn, Zn, Cd und Ag umfaßt. Diese Katalysatoren erlauben die Reduktion von substituierten Anilinen zu den zugehörigen Cyclohexylaminen; die entsprechenden Dicyclohexylamine fehlen jedoch völlig. Dies gilt gleichermaßen für Co-Katalysatoren, die einen basischen Zusatz enthalten (GB 969 542) sowie für Raney-Co (JP 68/03 180).

Bei den beschriebenen Druckhydrierungsverfahren von Anilin entsteht das Dicyclohexylamin neben dem Cyclohexylamin lediglich als Nebenprodukt oder überhaupt nicht. Um Dicyclohexylamin in größeren Mengen zu erhalten, wird es nach separaten Verfahren hergestellt. So kann es beispielsweise durch Druckhydrierung von Diphenylamin unter Verwendung eines Ru/Al₂O₃-Katalysators gewonnen werden (DE-B 1 106 319). Weiterhin entsteht Dicyclohexylamin bei der Umsetzung von Cyclohexanon mit Cyclohexylamin in Gegenwart von Pd auf Kohle unter einem Wasserstoffdruck von 4 bar (FR 1 530 477).

US 5 360 934 offenbart ein verbessertes Hydrierverfahren mittels dem aromatische Amine zu den entsprechenden am Ring hydrierten Verbindungen umgesetzt werden. Die Verbesserung beruht auf der Verwendung eines Katalysators, der Rhodium enthält, das auf einen Träger aus κ-, θ- oder δ-Al₂O₃ aufgebracht ist.

US 4 960 941 offenbart ein verbessertes Hydrierverfahren mittels dem aromatische Amine zu den entsprechenden am Ring hydrierten Verbindungen umgesetzt werden. Die Verbesserung beruht auf der Verwendung eines Katalysators, der Rhodium enthält, das auf einen Träger aus TiO₂ aufgebracht ist.

US 5 773 657 offenbart die Hydrierung aromatischer Verbindungen, die mindestens eine Aminogruppe am aromatischen Kern tragen. Das Verfahren wird bei Drücken über 50 bar, bevorzugt in einem Druckbereich von 150 bis 300 bar, durchgeführt.

US 5 023 226 bezieht sich auf Rutheniumkatalysatoren, die zusätzlich Palladium und/oder Platin enthalten und auf einen Al₂O₃- oder Aluminiumspinellträger aufgebracht sind, der mit Chrom- und Manganverbindungen behandelt wurde.

Aus EP-A 0 501 265 ist ein Verfahren bekannt, gegebenenfalls substituiertes Cyclohexylamin und gegebenenfalls substituiertes Dicyclohexylamin durch katalytische Hydrierung von gegebenenfalls substituiertem Anilin herzustellen, wobei ein Katalysator eingesetzt wird, der Ru, Pd oder ein Gemisch beider Metalle enthält, die auf einem Träger aus Niobsäure oder Tantalsäure oder auf einem Gemisch beider aufgebracht sind. Aus EP-A 503 347 ist ein weiteres Verfahren bekannt, gegebenenfalls substituiertes Cyclohexylamin und gegebenenfalls substituiertes Dicyclohexylamin durch Hydrierung eines entsprechend substituierten Anilins herzustellen, wobei ein Katalysator eingesetzt wird, bei dem ein α- oder γ-Al₂O₃ als Träger zunächst mit mindestens einer Verbindung von Seltenen Erdmetallen und mit mindestens einer Verbindung des Mangans behandelt wird und danach mit mindestens einer Pd-Verbindung behandelt wird.

Alle genannten Katalysatoren und Verfahren weisen jedoch immer noch Nachteile auf hinsichtlich Umsatz, Selektivität, Standzeit der Katalysatoren, notwendiger Mitverwendung von NH₃ usw. Gravierend wirkt sich in Katalysatorschüttungen für die kontinuierliche Rieselphasenhydrierung die bisher allen Ru enthaltenden Katalysatoren eigene Neigung aus, mit steigenden Temperaturen Desaminierungen und Hydrogenolyse der Moleküle bis zum Methan zu katalysieren. Die an sich schon exotherme Hydrierung kann also beispielsweise bei geringfügigen Abweichungen von einer gegebenen Temperatur zunächst langsam, dann gegebenenfalls sehr rasch in die weit stärker exotherme Methanisierung übergehen und zu einer nicht mehr beherrschbaren Situation bis hin zu Explosionen führen. Bei Verwendung von Ru enthaltenden Katalysatoren müssen daher sehr umfangreiche und zuverlässige Sicherheitsvorkehrungen getroffen werden. Damit aber ist die Eignung dieser Katalysatoren für technische Anlagen in Frage gestellt.

Welche Probleme auch heute trotz des erfolgten Fortschritts noch auftreten, zeigt die 1992 angemeldete EP-A 0 560 127: Die hier eingesetzten Ru-Pd-Katalysatoren auf alkalischen Trägern können zwar bei niedrigem Druck aromatische Amine hydrieren, doch dürfen sie nur gering mit 0,03 bis 0,05 g/ml Katalysator und Stunde belastet werden, was große Katalysatormengen und Reaktoren verlangt; NH₃ muß in großen Mengen zudosiert werden und die Temperaturen werden in der Nähe von 160°C gehalten. Dennoch erhält man bei weiterhin unvollständigem Umsatz noch immer Hydrogenolyse, erkennbar an der Bildung von Benzol und Cyclohexan; die Selektivität läßt zu wünschen übrig, und die Standzeit der Katalysatoren ist deutlich geringer als beispielsweise die in EP-A 0 324 983. Die beginnende Desaktivierung des Katalysators deutet sich durch den langsam fallenden Umsatz an.

Aufgabe der Erfindung war es deshalb, Katalysatoren für die technische Hydrierung aromatischer Amine zu cycloaliphatischen Aminen zur Verfügung zu stellen, die bei hoher Belastung einen vollständigen Umsatz bewirken, hohe Selektivität hinsichtlich der Bildung primärer und sekundärer cycloaliphatischer Amine besitzen, eine lange Lebensdauer aufweisen und vor allem keine Hydrogenolyse und Methanisierung der Substrate mehr auslösen.

In einem umständlichen Verfahren kann Dicyclohexylamin aus dem Hydrierprodukt des Anilins an einem Ni-Katalysator durch fraktioniertes Auskondensieren gewonnen werden. Aus dem zurückbleibenden Gemisch wird ein Teil des mitentstandenen Ammoniaks entfernt und der Rest wieder in die Reaktion zurückgeführt (DE-C 805 518).

In EP-A 0 208 933 werden Rh-Katalysatoren auf mit Cr-Mn-Salzen modifizierten Trägern beschrieben. Die Katalysatoren wurden zur Dehydrierung von Vorstufen zur o-Phenylphenol-Bildung bei hohen Temperaturen entwickelt.

EP-A 0 535 482 beschreibt ebenfalls temperaturresistente Rh-Katalysatoren auf mit Cr-Mn-Salzen modifizierten Trägern für die Herstellung von o-Phenylphenol. Die Kontakte enthalten dabei zusätzlich zum Rh weitere Edelmetalle.

Die Rh-Katalysatoren können in thermostatisierten stationären Katalysatorschüttungen bei niedrigen Drucken und Temperaturen zwischen 300 und 400°C zur Dehydrierung eingesetzt werden.

Überraschenderweise wurde gefunden, daß Kontakte die Rh auf speziell behandelten Trägermatrialien enthalten, potente Katalysatoren zur Realisierung eines Verfahrens zur Druckhydrierung von Anilinen sind, die auch bei hohen Temperaturen und Drucken keine Tendenz zur stark exothermen Hydrogenolyse zu Methan zeigen und damit eine hohe Produktionssicherheit gewährleisten.

Gegenstand der Erfindung ist ein Verfahren zur Hydrierung von aromatischen Aminen zu Gemischen aus cycloaliphatischen Aminen und dicycloaliphatischen Aminen bei Drucken zwischen 50 und 500 bar an mit Basen behandelten Edelmetallträgerkatalysatoren, dadurch gekennzeichnet, daß das Trägermaterial mit Salzen von Cr, Mo, W, Mn oder Re oder Gemischen solcher Salze belegt worden ist, und daß das so erhaltene Trägermaterial mit Rh als Edelmetall und gegebenenfalls zusätzlich mit Ir, Ru, Os, Pd und/oder Pt aktiviert worden ist.

Geeignete Ausgangsverbindungen für das erfindungsgemäße Verfahren sind aromatische Amine wie sie beispielsweise in DE-AS 2 502 894 und US 3 636 108 beschrieben sind. Bevorzugt sind Anilin, C₁-C₆-Alkylaniline sowohl im Kern wie am Stickstoff alkyliert, C₁-C₆-alkylierte Diaminobenzole, Aminonaphthaline und C₁-C₃-alkylierte Aminonaphthaline, Diaminonaphthaline und Diamino-diphenyl-C₁-C₃-alkane.

Genannt seien beispielsweise Anilin, N-Methylanilin, N-Ethylanilin, N,N-Dimethylanilin, N,N-Diethylanilin, N-Ethyltoluidin, N-Cyclohexylanilin, N-Cyclohexylidenanilin, o-, m-, p-Toluidin, 2,4-, 2,6-, 2,3-Diamino-toluol, Diphenylamin, 1- und 2- Aminonaphthalin, 1,4-, 1,5-, 2,5-, 2,6-, 2,7-Diaminonaphthalin und die isomeren Diaminophenylmethane.

Bevorzugt genannt seien beispielsweise Anilin, N-Methylanilin, N-Ethylanilin, N,N-Dimethylanilin, N,N-Diethylanilin, N-Cyclohexylanilin, N-Cyclohexylidenanilin, o-, m-, p-Toluidin, 2,4-, 2,6-, 2,3-Diamino-toluol, Diphenylamin.

Besonders bevorzugt genannt seien beispielsweise Anilin, 2,4- und 2,6-Diamino-toluol.

Ganz besonders bevorzugt wird das erfindungsgemäße Verfahren eingesetzt, um Anilin zu hydrieren.

Die Edelmetallträgerkatalysatoren für das erfindungsgemäße Verfahren bestehen aus Trägermaterial, das mit Salzen der Metalle Cr, Mo, W, Mn oder Re oder Gemischen solcher Salze belegt worden ist, ferner beinhalten die Edelmetallträgerkatalysatoren Rh als Edelmetall und gegebenenfalls eine zusätzliche Edelmetallkomponente der Reihe Ir, Ru, Os, Pd und/oder Pt.

Geeignete Trägermaterialien sind beispielsweise Tonerden, Al₂O₃ in den verschiedenen Modifikationen (α, κ, η, γ), weiter ansonsten für Edelmetalle übliche Trägermaterialien wie TiO₂, Kieselgur, Silicagel, BaCO₃, CaCO₃, ZnO, MgO, Bims, ZrO₂, Aktivkohle und die Oxide bzw. Oxidhydrate von Cr, Mo, W, Mn und/oder Re. Bevorzugte Trägermaterialien sind TiO₂, BaCO₂, MgO, besonders bevorzugt γ- Al₂O₃ und die Oxide bzw. Oxidhydrate von Cr, Mo, W, Mn und/oder Re, ganz besonders bevorzugt γ- Al₂O₃.

Das Trägermaterial kann als Pulver oder in stückiger Form als Kugeln oder als Extrudat wie Ringe, Wagenräder u.s.w. eingesetzt werden, ferner sind Formkörper wie z.B. Wabenkörper oder Kreuzkanalstrukturen verwendbar.

Vorzugsweise wird ein Trägermaterial mit großer BET-Oberfläche eingesetzt. Die BET-Oberfläche sollte oberhalb 50 m²/g, bevorzugt zwischen 100 und 500 m²/g, besonders bevorzugt zwischen 200 und 400 m²/g liegen.

Enthält das Trägermaterial Oxide oder Oxidhydrate von Cr, Mo, W, Mn oder Re oder Gemische solcher Oxide oder Oxidhydrate, kann gegebenenfalls auf die nachfolgend beschriebene Modifikation des Trägermaterials vor dem Aufbringen der Edelmetallkomponenten verzichtet werden.

Wird ein Cr, Mo, W, Mn oder Re-freies Trägermaterial eingesetzt, ist jenes zuerst mit einer oder meherer dieser Komponenten zu belegen. Dies kann z.B. durch Tränken oder Besprühen des Trägermaterials mit geeigneten Salzen dieser Elemente geschehen. Durch Trocknen und danach Tempern bei Temperaturen von ca. 200 bis 450°C werden die aufgebrachten Salze in auf dem Trägermaterial haftende Verbindungen überführt. Das Aufbringen der Verbindungen von Cr, Mo, W, Mn und/oder Re kann jedoch auch durch gemeinsames Ausfällen von Oxid-Hydroxid-Gemischen auf dem getränkten Trägermaterial mit Alkali-, Erdalkali-, oder Ammonium-Hydroxiden und gegebenenfalls anschließendes Auswaschen löslicher Anteile mit Wasser geschehen.

Besonders bevorzugt wird eine gleichmäßige Fällung durch langsame Freisetzung der Base durch Hydrolyse einer weniger basischen Vorstufe, besonders geeignet sind dazu Harnstoffe und Urethane, ganz besonders geeignet ist Harnstoff.

Das so vorbehandelte Trägermaterial wird getrocknet und dann zwischen 10 Minuten und 10 Stunden auf 200 bis 450°C, bevorzugt 250 bis 430°C erhitzt, wobei die Temperatur innerhalb dieses Bereiches auch nach und nach erhöht werden kann.

Geeignete Salze von Cr, Mo, W, Mn und Re sind beispielsweise die Acetate, Nitrate, Halogenide oder Sulfate. Ebenso geeignet sind die wasserlöslichen Oxide der höheren Oxidationsstufen, besonders die Ammoniumsalze der Cr, Mo, W, Mn und Re -Oxide.

Bevorzugt werden Trägermaterialien eingesetzt, die mit Cr- und/oder Mn- Salzen vorbehandelt worden sind.

Nach dem gegebenenfalls durchgeführten Auswaschen löslicher Verbindungen und der Trocknung und Temperung des mit Cr, Mo, W, Mn und/oder Re modifizierten Trägermaterials ist das Trägermaterial zur Aufnahme der übrigen Wirkstoffe bereit.

Die übrigen Wirkstoffe sind Rh und Alkali- oder Erdalkali-Hydroxid, gegebenenfalls Ir, Ru, Os, Pd und/oder Pt und gegebenenfalls Alkali- oder Erdalkalisulfat. Die Edelmetalle werden in Form von Lösungen ihrer Salze z.B. in Wasser aufgebracht. Als Salze sind z.B. geeignet die Halogenide, bevorzugt die Chloride, Acetate, Nitrate und Acetylacetonate. Als Alkalihydroxid ist z.B. NaOH oder KOH geeignet, als Erdalkalihydroxid z.B. Mg(OH)₂.

Als Sulfatkomponente ist z.B. K₂SO₄ zu nennen. Die Verbindungen können einzeln oder zusammen durch Tränken oder Besprühen aufgebracht werden. Zwischen jedem Tränkungsschritt erfolgt eine Trocknung.

Bevorzugt wird zuerst Rh, dann gegebenenfalls die Edelmetalle zur Modifizierung aufgebracht, gefolgt vom Alkalihydroxid und gegebenenfalls vom Alkalisulfat und gegebenenfalls einer weiteren Tränkung mit Base.

Nach jeder Tränkung mit Edelmetall wird gegebenenfalls mit z.B. Wasserstoff oder einem anderen Reduktionsmittel reduziert, in jedem Fall wird am Ende der letzten Trocknung z.B. mit Wasserstoff bei Temperaturen zwischen 80 und 350°C reduziert.

Der fertige Edelmetallträgerkatalysator enthält zwischen 0,1 und 10 Gew.-%, bevorzugt zwischen 0,3 und 3 Gew.-% Edelmetall, das zwischen 100 und 30%, bevorzugt zwischen 100 und 70% aus Rh besteht, das restliche Edelmetall besteht aus Ir, Ru, Os, Pd und/oder Pt. Ferner enthält der Katalysator zwischen 0,05 und 5 Gew.-% Cr, Mo, W, Mn und/oder Re, bevorzugt Cr und/oder Mn, weiterhin zwischen 0,05 und 15 Gew.-% Alkali- oder Erdalkalimetallionen und gegebenenfalls zwischen 0,05 und 3 Gew.-% Schwefel in Form von Verbindungen.

Bevorzugt wird im erfindungsgemäßen Verfahren ein geeigneter Edelmetallträgerkatalysator stückig in Form fester Schüttungen eingesetzt. Die Schüttungen können ohne Wärmeabfuhr sein oder durch Verwendung von mit Wärmeträger durchströmten oder umspülten Rohrbündeln thermostatisiert werden. Ebenso ist eine Kombination aus thermostatisierten und nicht thermostatisierten Schüttungen vorteilhaft, oder eine Folge von nicht thermostatisierten Reaktoren mit zwischengeschalteten Kühlem. Die Ausgestaltung geeigneter Reaktoren für solche Schüttungen ist Stand der Technik und dem Fachmann bekannt.

Die Umsetzung kann in der Form erfolgen, daß aromatisches Amin und Wasserstoff gegebenenfalls zusammen mit zu recyclisierenden Verbindungen, wie z.B: Wasserstoff, Ammoniak, N-Cyclohexylanilin erwärmt werden, die erwärmte Mischung über den Kontakt gefahren wird, ein Teil der kondensierbaren Verbindungen durch Abkühlen niedergeschlagen und zusammen mit der flüssigen Phase ausgeschleust werden, vom verbleibenden Gasstrom ein Teil zur Ausschleusung von inerten Verbindungen abgezweigt und der Rest durch Verdichtung der Reaktion wieder zugeführt wird.

Das erfindungsgemäße Verfahren kann beispielsweise bei Temperaturen zwischen 100 und 400°C, bevorzugt zwischen 150 und 350°C, besonders bevorzugt zwischen 170 und 330°C durchgeführt werden.

Die Umsetzung erfolgt in einem Druckbereich zwischen 50 und 500 bar, bevorzugt zwischen 100 und 400 bar, besonders bevorzugt zwischen 200 und 300 bar.

Das umzusetzende aromatische Amin kann beispielsweise zusammen mit Wasserstoff im molaren Verhältnis zwischen 1:500 und 1:5, bevorzugt zwischen 1:200 und 1:10, besonders bevorzugt zwischen 1:150 und 1:40 umgesetzt werden.

Zusammen mit den aromatischen Aminen und dem Wasserstoff können geringe Mengen Ammoniak über den Kontakt gefahren werden.

Die Belastung der Kontakte im erfindungsgemäßen Verfahren kann zwischen 0,05 und 5 kg, bevorzugt zwischen 0,2 und 2 kg Anilin pro Liter Katalysator und Stunde liegen.

Die Selektivitäten bezüglich Cyclohexylamin und Dicyclohexylamin im erfindungsgemäßen Verfahren liegen deutlich über 90%, unterhalb 300°C über 98%, unterhalb 250°C über 99%.

Das Verhältnis zwischen Monocyclohexylamin und Dicyclohexylamin variiert zwischen 20:1 und 0,8:1, je nach Basenmenge womit der Kontakt nachbehandelt worden ist, Reaktionstemperatur und Ammoniakgehalt der Eduktmischung.

Das erfindungsgemäße Verfahren ermöglicht aromatische Amine flexibel in Gemische aus Mono- und Dicyclohexylamin zu überführen, dabei kann der Katalysator hoch belastet werden und die Reaktion ist aufgrund hoher Selektivitäten auch bei hohen Temperaturen thermisch sicher zu führen!

### Beispiele

### Beispiel 1 (Katalysatorpräparation)

1 L γ-Al₂O₃ der Firma Rhone-Poulenc (SPH 501, Kugeln, ⌀ = 4-6 mm, BET-Oberfläche ca. 350 m²/g) wurden mit 320 ml einer Lösung aus 30,1 g MnSO₄ × H₂O, 22,3 g (NH₄)₂Cr₂O₇ und 164 g Harnstoff getränkt. Der getränkte Träger wurde 1 h bei 90°C in einer gesättigten Wasserdampfatmosphäre bewegt. Danach erfolgten zwei Wäschen mit je 160 ml Wasser zur Entfernung löslicher Verbindungen. Der so erhaltene Träger wurde getrocknet und anschließend 30 Minuten bei 300°C in einer Drehtrommel getempert.

20,3 g RhCl₃ in 360 ml Wasser wurden durch Tränkung aufgebracht und der Katalysatorvorläufer anschließend bei 110°C getrocknet.

Danach wurden 320 ml einer Lösung aus 24 g NaOH und 24 g K₂SO₄ in Wasser aufgebracht, getrocknet und nochmals mit 50 g NaOH in 320 ml Wasser getränkt.

Der Kontakt wurde getrocknet und 3,5 h bei 160°C im Wasserstoffstrom aktiviert.

Der fertige Kontakt enthält 8 g Rh, 9,2 g Cr, 9,8 g Mn, 74 g NaOH und 24 g K₂SO₄ pro Liter.

### Beispiel 2

50 ml Katalysator aus Beispiel 1 wurden mit einer Schütthöhe von 30,5 cm in ein druckfestes Reaktorrohr mit einem Innendurchmesser von 1,8 cm gegeben. Der Reaktor war ölbeheizt und wurde bei einem Wasserstoffdruck von 270 bar betrieben. Vor Reaktionsbeginn wurde der Katalysator 21 h bei 141°C unter Druck im Wasserstoffstrom aktiviert.

Die nachfolgende Tabelle zeigt die Zusammensetzung des Produktgemisches bei einer Belastung von 1 kg Anilin pro L Katalysator und Stunde und einem molaren Verhältnis von Wasserstoff zu Anilin von 90/1.

Der Katalysator wurde 2000 h ohne Desaktivierungsanzeichen betrieben, dann wurde der Versuch abgebrochen.

**Tabelle**

| **Anilin-Hydrierung unter Druck, ohne Methanbildung auch bei hohen Temperaturen.** | | | | | | | | |
|---|---|---|---|---|---|---|---|---|
| Temp. °C | CHA/DCA | CHA % | DCA % | ANI % | Bz % | CYOL % | Rest % | CH₄ vpm |
| 180 | 13,4 | 91,2 | 6,8 | 1,46 | 0,06 | 0,28 | 0,20 | 0 |
| 220 | 9,03 | 89,4 | 9,9 | 0 | 0,13 | 0,30 | 0,27 | 0 |
| 300 | 1,14 | 52,0 | 45,6 | 0 | 1,89 | 0,25 | 0,26 | 5 |
| 330 | 1,36 | 52,5 | 38,6 | 0 | 8,07 | 0,27 | 0,56 | 12 |

Die Spaltenbezeichnungen bedeuten von links nach rechts:
Temperatur des Thermostatisierten Ölkreislaufes und der Edukte in °C,
Gewichtsverhältnis von Cyclohexylamin (CHA) zu Dicyclohexylamin (DCA) im flüssigen (fl.) Produkt,
Gewichtsprozente Cyclohexylamin im fl. Produkt,
Gewichtsprozente Dicyclohexylamin im fl. Produkt,
Gewichtsprozente unumgesetztes Anilin (ANI) im fl. Produkt,
Gewichtsprozente Benzol (Bz) im fl. Produkt
Gewichtsprozente Cyclohexanol (CYOL) im fl. Produkt,
Gewichtsprozente der Summe der restlichen Verbindungen im fl. Produkt,
Volumenteile Methan pro 1 Mio. Volumenteilen Abgas.
Cyclohexanol entsteht aufgrund geringer Mengen Wasser im Wasserstoffgas.

## Patentansprüche

1. Verfahren zur Hydrierung von aromatischen Aminen zu einem Gemisch aus cycloaliphatischen Aminen und dicycloaliphatischen Aminen in variablen Mengenverhältnissen bei Drucken zwischen 50 und 500 bar an mit Basen behandelten Edelmetallträgerkatalysatoren, dadurch gekennzeichnet, daß das Trägermaterial des Edelmetallträgerkatalysators mit Salzen von Metallen der Reihe Cr, Mo, W, Mn oder Re oder Gemischen solcher Salze belegt worden ist, und daß das so erhaltene Trägermaterial mit Rh als Edelmetallkomponente aktiviert worden ist.

2. Verfahren gemäß Anspruch 1, dadurch gekennzeichnet, daß das Trägermaterial zusätzlich zum Rh mit einer Edelmetall der Reihe Ir, Ru, Os, Pd und/oder Pt aktiviert worden ist.

3. Verfahren nach Anspruch 1, dadurch gekennzeichnet, daß der eingesetzte Edelmetallträgerkatalysator zwischen 0,1 und 10 Gew.-% Edelmetall enthält.

4. Verfahren gemäß Anspruch 3, dadurch gekennzeichnet, daß die 0,1 bis 10 Gew.-% Edelmetall zwischen 100 und 30 % aus Rh bestehen.

5. Verfahren gemäß der Ansprüche 1 bis 4, dadurch gekennzeichnet, daß der Edelmetallträgerkatalysator zwischen 0,05 und 5 Gew.-% Cr, Mo, W, Mn und/oder Re, weiterhin zwischen 0,05 und 15 Gew.-% Alkali- oder Erdalkalimetallionen und gegebenenfalls 0,05 und 3 Gew.-% Schwefel in Form von Verbindungen enthält.

6. Verfahren gemäß der Ansprüche 1 bis 5, dadurch gekennzeichnet, daß die Edelmetallträgerkatalysatoren zwischen 0,05 und 5 Gew.-% Cr und/oder Mn enthalten.

7. Verfahren gemäß der Ansprüche 1 bis 5, dadurch gekennzeichnet, daß die Umsetzung bei Temperaturen zwischen 100° und 400°C durchgeführt wird.

8. Verfahren gemäß der Ansprüche 1 bis 7, dadurch gekennzeichnet, daß als aromatische Amine Anilin, C₁-C₆-Alkylaniline sowohl im Kern wie am Stickstoff alkyliert, C₁-C₆-alkylierte Diaminobenzole, Aminonapthaline und C₁-C₃-alkylierte Aminonaphthaline, Diaminonaphthaline und Diamino-diphenyl- C₁-C₃-alkane eingesetzt werden.

9. Verfahren gemäß der Ansprüche 1 und 2, dadurch gekennzeichnet, daß als Trägermaterial Tonerden, Al₂O₃ in den verschiedenen Modifikationen (α, κ, η γ), ansonsten für Edelmetalle übliche Trägermaterialien wie TiO₂, Kieselgur, Silicagel, BaCO₃, CaCO₃, ZnO, MgO, Bims, ZrO₂, Aktivkohle oder die Oxide bzw. Oxidhydrate von Cr, Mo, W, Mn und/oder Re eingesetzt werden.

10. Verfahren gemäß Anspruch 9, dadurch gekennzeichnet, daß das Trägermaterial als Pulver oder in stückiger Form als Kugeln oder als Extrudat wie Ringe, Wagenräder oder Formkörper eingesetzt wird.

11. Verfahren zum Herstellen von Cr, Mo, W, Mn oder Re-haltigen Edelmetallträgerkatalysatoren, gekennzeichnet durch die Schritte
i) Tränken oder Besprühen des Trägermaterials mit geeigneten Salzen der Elemente Cr, Mo, W, Mn oder Re,
ii) Trocknen und Tempern bei Temperaturen von ca. 200 bis 450°C,
iii) Auswaschen löslicher Verbindungen,
iv) Aufnahme von Rh und gegebenenfalls Ir, Ru, Os, Pd und/oder Pt,
v) Aufnahme von Alkali- oder Erdalkali-Hydroxid und gegebenenfalls Alkali- oder Erdalkalisulfat,
vi) abschließende Trocknung und Reduktion mit Wasserstoff bei Temperaturen zwischen 80 und 350°C.

12. Edelmetallträgerkatalysatoren, erhältlich durch
i) Tränken oder Besprühen des Trägermaterials mit geeigneten Salzen der Elemente Cr, Mo, W, Mn oder Re
ii) Trocknen und Tempern bei Temperaturen von ca. 200 bis 450°C,
iii) Auswaschen löslicher Verbindungen,
iv) Aufnahme von Rh und gegebenenfalls Ir, Ru, Os, Pd und/oder Pt,
v) Aufnahme von Alkali- oder Erdalkali-Hdroxid und gegebenenfalls Alkali- oder Erdalkalisulfat,
vi) abschließende Trocknung und Reduktion mit Wasserstoff bei Temperaturen zwischen 80 und 350°C.

13. Verwendung von Edelmetallträgerkatalysatoren gemäß Anspruch 12 bei der Herstellung variabler Gemische aus Cyclohexylamin und Dicyclohexylamin.
